# EUROPEAN PATENT SPECIFICATION

(11) **EP 0 702 015 B1**
(45) Date of publication and mention of the grant of the patent: **24.09.1997**
(21) Application number: 95810561.1
(22) Date of filing: 11.09.1995
(51) Int. Cl.: C07D 498/04, C07D 513/04, A61K 31/42, A61K 31/425

(54) **Bicyclic oxazole and thiazole substituted ethers**
Substituierte bizyklische Oxazol- und Thiazol-Äther
Ethers substitués d'oxazole et thiazole bicycliques

(30) Priority: 13.09.1994 US 305249
(43) Date of publication of application: 20.03.1996
(73) Proprietor: Novartis AG, 4058 Basel (CH); SANDOZ-PATENT-GMBH, 79539 Lörrach (DE); SANDOZ-ERFINDUNGEN Verwaltungsgesellschaft m.b.H., 1235 Wien (AT)
(72) Inventor: Aicher, Thomas D., Cedar Knolls, NJ 07927 (US); Cheon, Seung Hoon, Glen Ridge, NJ 07028 (US); Nadelson, Jeffrey, Denville, NJ 07834 (US); Simpson, William Ronald James, Mendham, NJ 07945 (US); Houlihan, William Joseph, Mountain Lakes, NJ 07046 (US)

(56) References cited:
- EP-A- 0 590 793
- WO-A-94/22857
- FR-A- 2 554 814
- US-A- 5 321 036

## Description

The invention relates to bicyclic oxazole and thiazole substituted ethers. It concerns the compounds of formula I 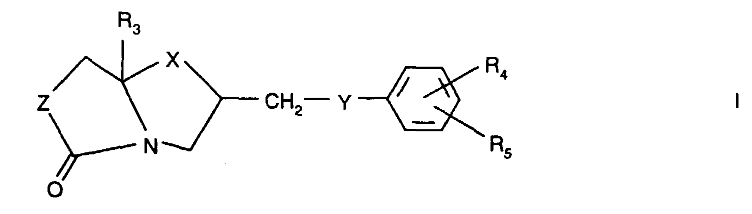 where
- X and Y: are each independently oxygen or sulfur;
- Z: is -CR₁R₂- or -CR₁R₂CH₂- where R₁ and R₂ are each independently hydrogen or lower alkyl;
- R₃: is phenyl optionally mono- or disubstituted independently by halo, lower alkyl, lower alkoxy or trifluoromethyl; or is biphenyl; phenoxyphenyl; naphthyl; 9,10-dihydrophenanthryl; or pyridyl; and
- R₄ and R₅: are each independently hydrogen, halo, lower alkyl, lower alkoxy, trifluoromethyl or -COOR₆ where R₆ is hydrogen, lower alkyl or tri(lower)alkylsilyl(lower)alkyl;
in free form or salt form as appropriate,
hereinafter briefly named "the compounds of the invention".

Lower alkyl and lower alkoxy contain 1 to 4 carbon atoms and are especially methyl and methoxy. Halo can be fluoro, chloro, bromo, or iodo, preferably fluoro or chloro, especially chloro. X and Y are preferably oxygen. Z is preferably -CR₁R₂-. R₁ and R₂ are preferably hydrogen or methyl, especially hydrogen. R₃ is preferably phenyl mono- or disubstituted by chloro, especially 4-chloro or, even more preferably, 3,4-di-chloro. R₄ and R₅ are preferably hydrogen or chloro. R₄ is especially chloro, preferably 4-chloro. R₅ is especially hydrogen. R₆ is preferably lower alkyl, especially methyl.

When a phenyl ring is monosubstituted it preferably is substituted in the 4 position. When it is disubstituted it preferably is substituted in the 3 and 4 positions. Biphenyl preferably is 4-biphenyl. Phenoxyphenyl preferably is 4-phenoxyphenyl. Naphthyl preferably is 2-naphthyl. 9,10-Dihydrophenanthryl preferably is 9,10-dihydrophenanthren-2-yl. Pyridyl preferably is 4-pyridyl. Tri(lower)alkylsilyl(lower)alkyl preferably is (2-trimethylsilyl)ethyl.

The compounds of the invention may exist in free form or, particularly when R₆ is hydrogen, in salt form; when R₆ is hydrogen they preferably are in salt, especially alkali metal salt form. Preferred salts are the sodium or potassium, preferably the sodium salt. A compound of the invention in free form may be converted into a salt form as appropriate in conventional manner and vice-versa, e.g., when R₆ is hydrogen, by reacting the free acid with an alkali metal base, such as sodium methylate for the preparation of the sodium salt form.

Preferred compounds of the invention are the compounds of formula I where
- X and Y: are both oxygen;
- Z: is -CR₁R₂- as defined above;
- R₃: is phenyl optionally mono- or disubstituted independently by halo, lower alkyl, lower alkoxy or trifluoromethyl; or is 4-biphenyl; 4-phenoxyphenyl; 2-naphthyl; 9,10-dihydrophenanthryl; or 4-pyridyl;
- R₄: is hydrogen, halo, lower alkyl, lower alkoxy, trifluoromethyl or -COOR₆ₐ where R₆ₐ is an alkali metal anion or lower alkyl; and
- R₅: is hydrogen or halo;
hereinafter briefly named "**compounds Ia**".

Especially preferred compounds of the invention are the trans diastereoisomers of the compounds of formula I where
- X and Y: are both oxygen;
- Z: is -CH₂- or -C(CH₃)₂-;
- R₃: is phenyl optionally mono- or disubstituted independently by fluorine or chlorine, or disubstituted by fluorine or chlorine and trifluoromethyl; or is 4-biphenyl; 4-phenoxyphenyl; 2-naphthyl; 9,10-dihydrophenanthryl; or 4-pyridyl;
- R₄: is 4-chloro; and
- R₅: is hydrogen;
hereinafter briefly named "**compounds Ib**".

A further group of compounds of the invention is the compounds of formula I where X and Y are as defined above;
- Z: is -CH₂-, -C(CH₃)₂- or -CH₂CH₂-;
- R₃: is phenyl optionally mono- or disubstituted independently by halo, methoxy or trifluoromethyl; 4-phenoxyphenyl; naphthyl; 9,10-dihydrophenanthryl; or pyridyl;
- R₄: is hydrogen; halo; methoxy; trifluoromethyl; or COOR₆ where R₆ is hydrogen, methyl or (2-trimethylsilyl)ethyl; and
- R₅: is hydrogen or halo;
in free form or salt form as appropriate, hereinafter briefly named "**compound Is**".

A further group of compounds of the invention is the compounds of formula I as defined above under formula I, in free form or alkali metal salt form as appropriate, hereinafter briefly named "**compounds Ip**".

The compounds of the invention may be prepared by a process which comprises
a) for the preparation of a compound of formula I where R₆ is other than hydrogen, reacting a compound of formula II 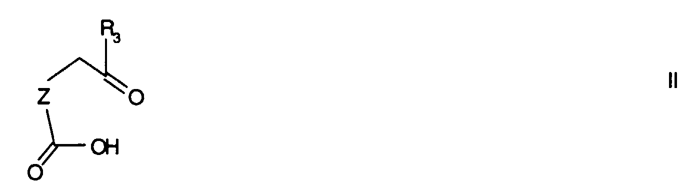 where R₃ and Z are as defined above,
   with a compound of formula III 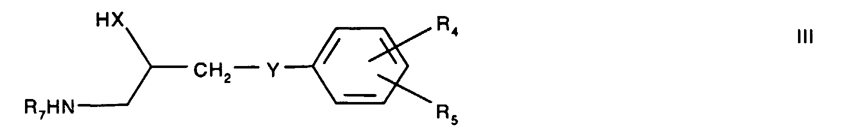 where X, Y, R₄ and R₅ are as defined above and
   R₇ is hydrogen or an amine protecting group; or
b) for the preparation of a compound of formula I where R₆ is hydrogen, hydrolyzing a compound of formula I where R₆ is lower alkyl or tri(lower)alkylsilyl(lower)alkyl,
and recovering the resultant compounds of formula I in free form or salt form as appropriate.

The process of the invention can be effected in conventional manner.

Process variant a) is a coupling reaction. It is effected preferably in an inert solvent such as toluene and in the presence of an organic acid such as p-toluene sulfonic acid or trifluoroacetic acid. When R₇ is a protecting group such as tert-butoxycarbonyl, trifluoroacetic acid is the preferred organic acid. The reaction is preferably run at a temperature between about 60°C to about 125°C, especially at the reflux temperature of the reaction mixture.

Process variant b) is a hydrolysis reaction. It is effected with an appropriate hydrolyzing agent such as tetrabutylammonium fluoride. A suitable solvent is e.g. tetrahydrofuran. Temperature is e.g. from about 0° to the boiling temperature of the reaction mixture, preferably about room temperature.

The compounds of the invention can be isolated from the reaction mixture and purified by conventional techniques, for example, flash chromatography and recrystallization.

As appears from formula I, the compounds of the invention can exist in the form of isomers, which can be prepared as such or readily separated and recovered by conventional techniques such as described below from isomeric mixtures. All such isomeric forms are included in the scope of this invention.

Thus the compounds can exist in the form of cis or trans isomers with respect to the asymetrically substituted ring carbon atoms carrying the -CH₂-Y- moiety in 2 position and the R₃ moiety in 7a or 8a position, respectively. The trans isomers are preferred. Each of the cis and trans isomers can have two optical configurations. The compounds may thus exist in the form of the cis or the trans racemate, or of the individual cis or the individual trans enantiomers.

The starting materials can also be prepared in conventional manner.

The compounds of formula III in which X is oxygen and R₇ is hydrogen may e.g. be prepared by reacting a compound of formula IV 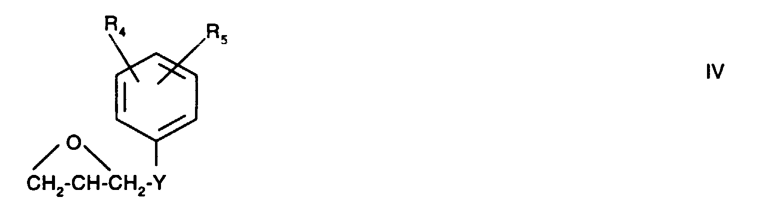 where Y, R₄ and R₅ are as defined above,
with aqueous ammonium hydroxide or condensed ammonia, preferably in an inert solvent such as methanol. The procedure may be carried out at about 30°C with aqueous ammonium hydroxide and from about room temperature to about 50°C with condensed ammonia, followed by isolation by conventional techniques, for example, evaporation or lyophilization.

The compounds of formula (III) in which X is sulfur may be prepared using a conventional Mitsunobo procedure in accordance with the following reaction scheme: 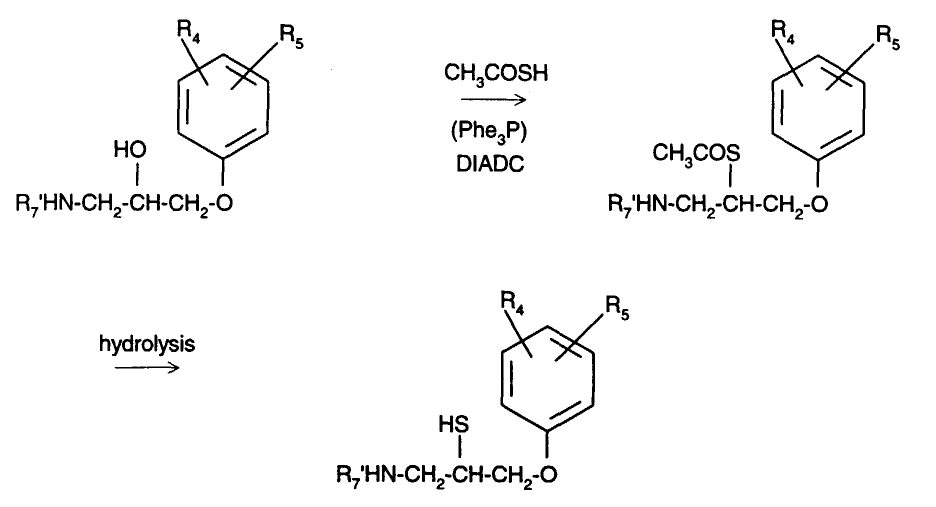 where R₄ and R₅ are as defined above; Phe is phenyl; R₇' is an amino protecting group; and DIADC is diisopropyl azodicarboxylate.

Isolation is e.g. by flash chromatography.

Many of the compounds of formula II and IV are known and may be prepared by methods described in the literature. The starting materials not specifically disclosed herein or in the literature may be prepared by conventional methods or as described in the Examples using known starting materials.

The following Examples illustrate the invention. They are not limitative. All temperatures are in degrees Centigrade.

### Example 1: (-)-trans-2S-[(4-Chlorophenoxy)methyl]-7A-(3,4-dichlorophenyl)-2,3,7,7a-tetrahydropyrrolo[2.1-b]oxazol-5(6H)-one

### [Formula I: X = Y = O; Z = CH₂; R₃ = 3,4-dichlorophenyl; R₄ = 4-Cl; R₅ = H; R₃ and -CH₂Y- carrying chain in trans to each other]

### [Process variant a)]

A mixture of 6.2 g of **3-(3,4-dichlorobenzoyl)propionic acid** (compound of formula II), 5 g of **(-)-3-(4-chlorophenoxy)-2S-hydroxypropylamine** (compound of formula III) and 0.05 g of p-toluenesulfonic acid monohydrate in 100 ml of toluene in a round bottom flask equipped with a Dean-Stark water separator is heated at reflux for 5 hours. After cooling the reaction mixture to room temperature, the solvent is evaporated in vacuo to yield a brown gum which is flash chromatographed on 250 g of silica using 1000 ml of hexane/ethyl acetate (2:1) to give 3.2 g of gum. The gum is crystallized from ethyl acetate and hexane to yield 2.5 g of **title compound** as a white solid [m.p. 102-104°; [α]_{D}²⁵ = -8.01° (c=1, MeOH)].

The starting material is obtained as follows:

### Method 1:

Step A: To a solution of 35.1 g of **triphenylphosphine** in 75 ml of tetrahydrofuran is added dropwise a solution of 21.6 ml of **diethyl azodicarboxylate** in 25 ml of tetrahydrofuran. As a precipitate begins to form in the reaction mixture, a solution of 10 g of **R(+)-glycidol** and 17.3 g of **p-chlorophenol** in 10 ml of tetrahydrofuran is added; the mixture is stirred at room temperature overnight. The solvent is evaporated under reduced pressure, and the solid residue obtained is taken up in a mixture of 250 ml of hexane and 250 ml of diethyl ether. After removing the insoluble material by filtration and washing with a mixture of 250 ml of hexane and 250 ml of diethyl ether, the combined organic phase is evaporated in vacuo to give an oil which is distilled to yield **4-chlorophenyl-S(+)-glycidyl ether** as an oil [b.p. = 112°/1.5 mmHg; [α]_{D}²⁵ = +4.81° (c=1, CHCl₃)].

Step B: A solution of 15.5 g of **4-chlorophenyl-S(+)-glycidyl ether** in 25 ml of methanol is added to 100 ml of ammonia in a sealable bomb at -78°. The sealed bomb is heated at 40° for 18 hours on an oil bath, then cooled to -78° and opened. The solvent is removed to yield **(-)-3-(4-chlorophenoxy)-2S-hydroxypropylamine** as a white solid [m.p. 107-111°; [α]_{D}²⁵ = -4.67° (c=1, CHCl₃)].

### Method 2:

To a solution of 4.5 g of **L(-)-dibenzoyltartaricacid** in 10 ml of methanol is added a solution of 5 g of **(±)-3-(4-chlorophenoxy)-2-hydroxypropylamine** in 10 ml of methanol. The flask is rinsed with 5 ml of methanol and added to the salt mixture. The salt mixture is cooled to 0° to start crystallization. After 3 hours, the salt mixture is filtered to yield 4.9 g of solid. This is dissolved in 58 ml of methanol and filtered, and then about 50 ml of diethyl ether is added to the cloud point. Rapid crystallization occurs at room temperature yielding 4.2 g of product. The recrystallization is repeated using a minimum of hot methanol and diethyl ether to obtain a white solid [m.p. 187-188°; [a]_{D}²⁵ = -72.9° (c=1, CH₃OH)]. This salt is dissolved in 2N NaOH, and the aqueous layer is extracted with methylene chloride (3 x 50 ml). The combined organic extracts are dried over anhydrous MgSO₄, filtered, and evaporated in vacuo to yield **(-)-3-(4-chlorophenoxy)-2S-hydroxypropylamine** [m.p. 107-111°; [a]_{D}²³ = -3.7° (c=1, CH₃OH)].

### Example 2: (+)-trans-2R-[(4-Chlorophenoxy)methyl]-7a-(3,4-dichlorophenyl)-2,3,7,7a-tetrahydropyrrolo[2,1-b]-oxazol-5(6H)-one

### [Diastereoisomer of the compound of Example 1]

### [Process variant a)]

Reaction of **(+)-3-(4-chlorophenoxy)-2R-hydroxypropylamine** in a manner analogous to Example 1 gives the **title compound** as a white solid [m.p. 102-104°; [α]_{D}²⁵ = +7.97° (c=1, MeOH)].

The starting material is obtained analogously as described in Example 1, using either method 1, starting from **(S)(-)-glycidol**, whereby step A gives **4-chlorophenyl-S(-)-glycidyl ether** as an oil [α]_{D}²⁵ = -3.9° (c=1, CHCl₃)] and step B gives **(+)-3-(4-chlorophenoxy)-2R-hydroxypropylamine** as a white solid [m.p. 104-107°; [α]_{D}²⁵ = +5.77° (c=1, CHCl₃)], or method 2 starting from an equivalent amount of **D(+)-dibenzoyltartaric acid** in place of L(-)dibenzoyltartaric acid, whereby there is obtained **(+)-3-(4-chlorophenoxy)-2R-hydroxypropylamine**[m.p. 108-110°; [α]_{D}²³ = +3.6° (c=1, CH₃OH)].

### Example 3: (±)-trans-2-[(4-Chlorophenoxy)methyl]-7a-(3,4-dichlorophenyl)-2,3,7,7a-tetrahydropyrrolo[2,1-b]oxazol-5(6H)-one and (±)-cis-2-[(4-chlorophenoxy)methyl]-7a-(3,4-dichlorophenyl)-2,3,7,7a-tetrahydropyrrolo[2,1-b]oxazol-5(6H)-one

### [Racemate of the enantiomers of Examples 1 and 2: R₃ and -CH₂Y-carrying chain in trans to each other; and corresponding cis racemate]

### [Process variant a)]

A mixture of 24.5 g of **3-(3,4-dichlorobenzoyl)propionic acid** and 20 g of **3-(4-chlorophenoxy)-2-hydroxypropylamine** is reacted analogously as described in Example 1. Flash chromatography is effected on 750 g of silica using 5000 ml of methylene chloride/methanol (99.5 : 0.5) as eluant to give a fast moving product and a slow moving product. The gum obtained after evaporation of the solvent from the faster moving product is crystallized from methylene chloride and diethyl ether to yield the **first title compound** (m.p. 126°). The **second title compound** is obtained as the slower moving cis product in a similar manner.

The starting material is obtained analogously as described in Example 1, method 1, step B, starting from 4-chlorophenyl glycidyl ether, whereby aqueous ammonium hydroxide solution at 30° is used in place of ammonia at -78°, to give **3-(4-chlorophenoxy)-2-hydroxypropylamine** as a white solid (m.p. 85-87°).

### Example 4: (±)-trans-2-[(4-Carboxyphenoxy)methyl]-7a-(4-chlorophenyl)-2,3,7,7a-tetrahydropyrrolo[2,1-b]oxazol-5(6H)-one

### [Formula I: X = Y = O; Z = CH₂; R₃ = 4-chlorophenyl; R₄ = 4-COOH; R₅ = H; racemate; R₃ and -CH₂Y- carrying chain in trans to each other; and corresponding sodium salt]

### [Process variant b) + salt formation]

To a solution of 8.0 g of **(±)-trans-2-{[4-[(2-trimethylsilylethoxy)carbonyl]phenoxy]methyl}- 7a-(4-chlorophenyl-2,3,7,7a-tetrahydropyrrolo[2,1-b]oxazol-5(6H)-one** (compound of Example 51) in 100 ml of tetrahydrofuran is added dropwise a solution of 38 ml of 1M tetrabutylammonium fluoride in tetrahydrofuran; and the mixture is stirred at room temperature for 16 hours. After evaporating the reaction mixture to dryness on a rotavapor, the gum obtained is dissolved in ethyl acetate, and the organic solution is washed with 10 ml of ice cold 0.5 N HCl, water, brine, dried over anhydrous magnesium sulfate and filtered. The filtrate is concentrated under reduced pressure to give the **title compound in free acid form** as a crystallline solid:
- ¹H-NMR [CDCl₃, (CD₃)₂SO]:: 2.12 ppm(1H,m); 2.45-2.68 (2H,m); 2.78 (1H,m); 3.17 (1H,dd); 4.02 (2H,m); 4.18 (1H,dd); 4.39 (1H,m); 6.95 (2H,d); 7.36 (2H,d); 7.43 (2H,d); 7.93 (2H,d); 12.10 (1 H,bs).

This acid is dissolved in a mixture of 40 ml of methanol and 10 ml of methylene chloride and then treated with 1.8 ml of 1 M sodium methoxide in methanol for 5 minutes at room temperature. The mixture is concentrated under reduced pressure and the residue obtained is triturated with diethyl ether to yield the **title compound in sodium salt form** as a white solid (m.p. > 200°).

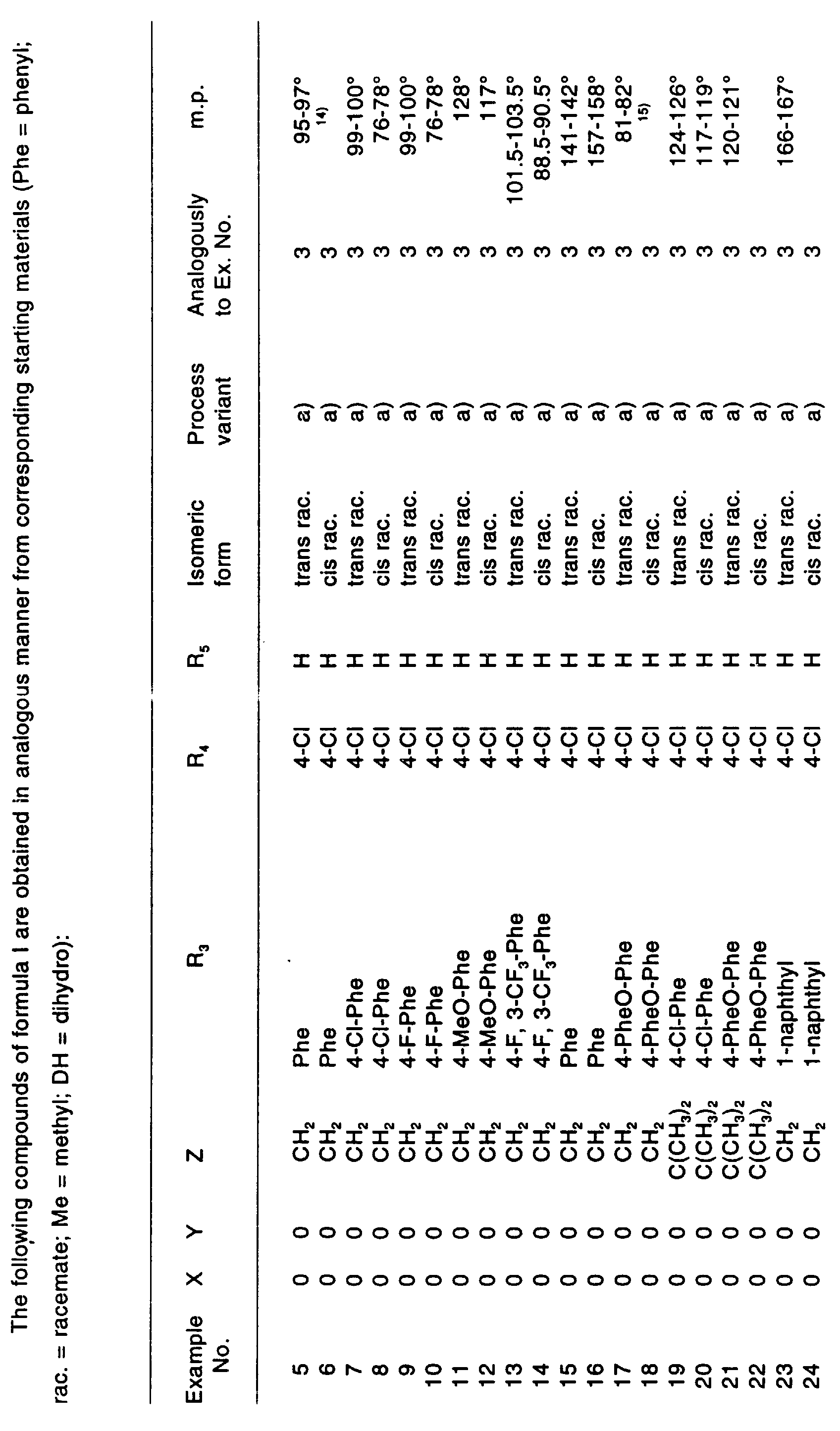

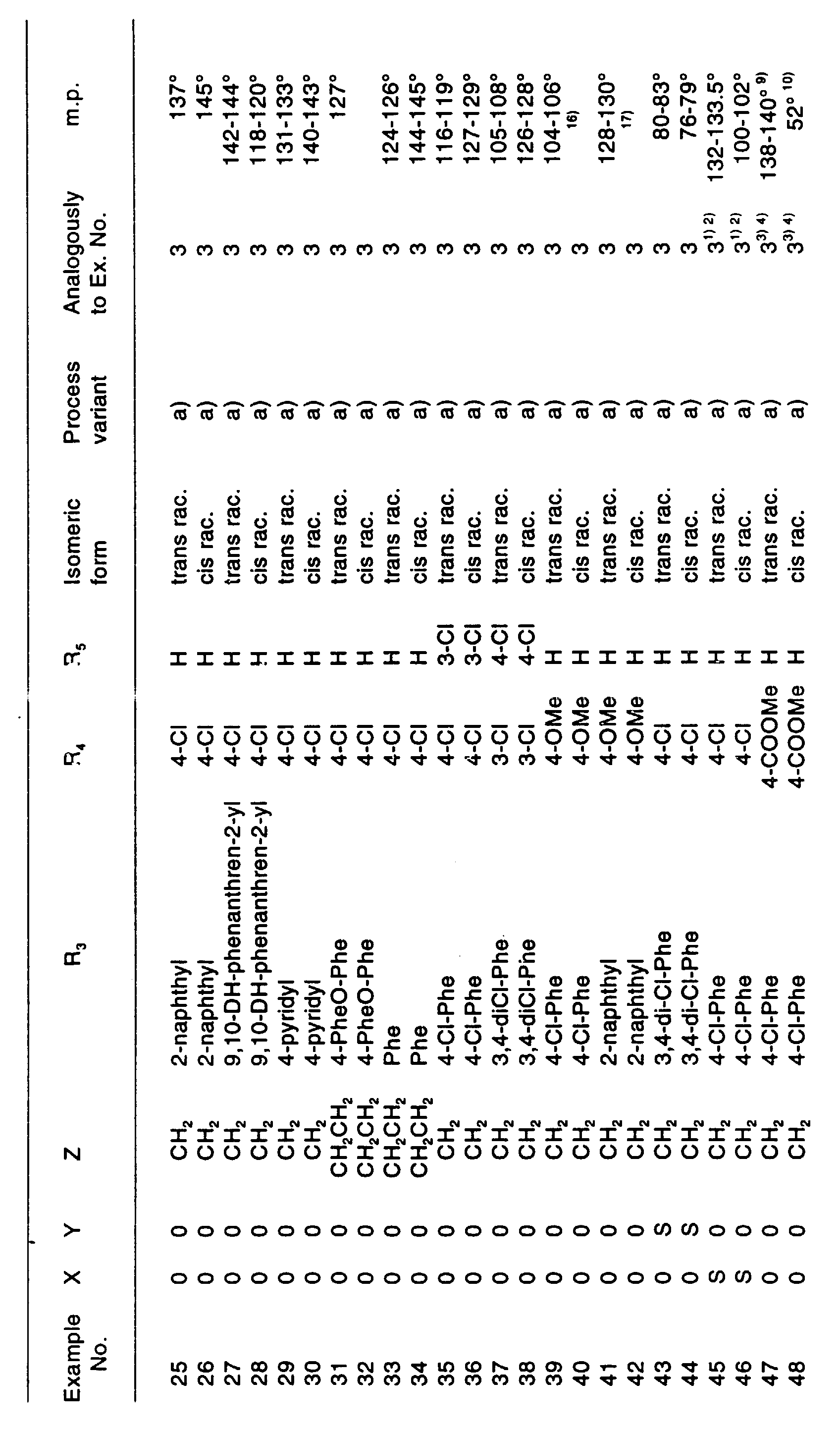

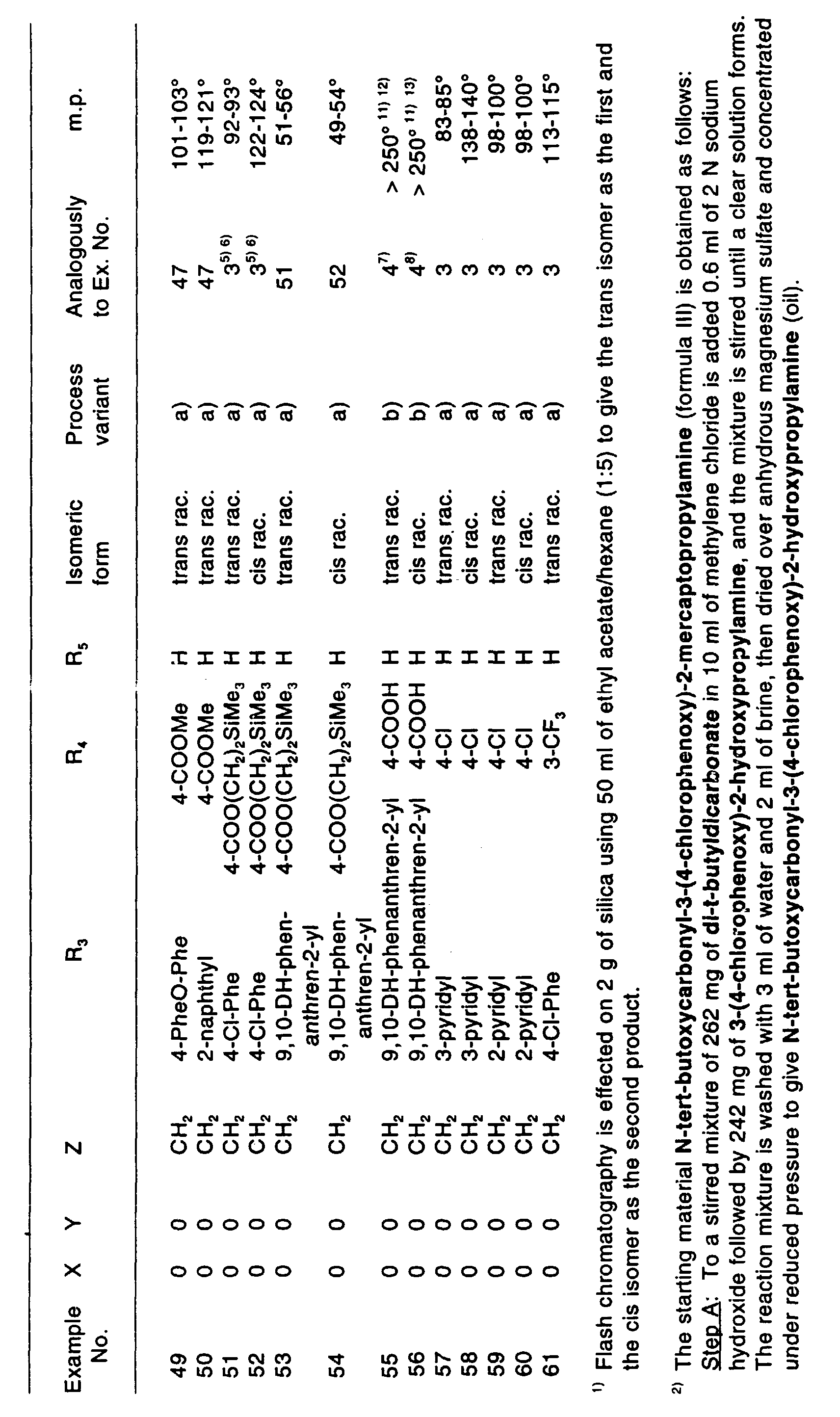

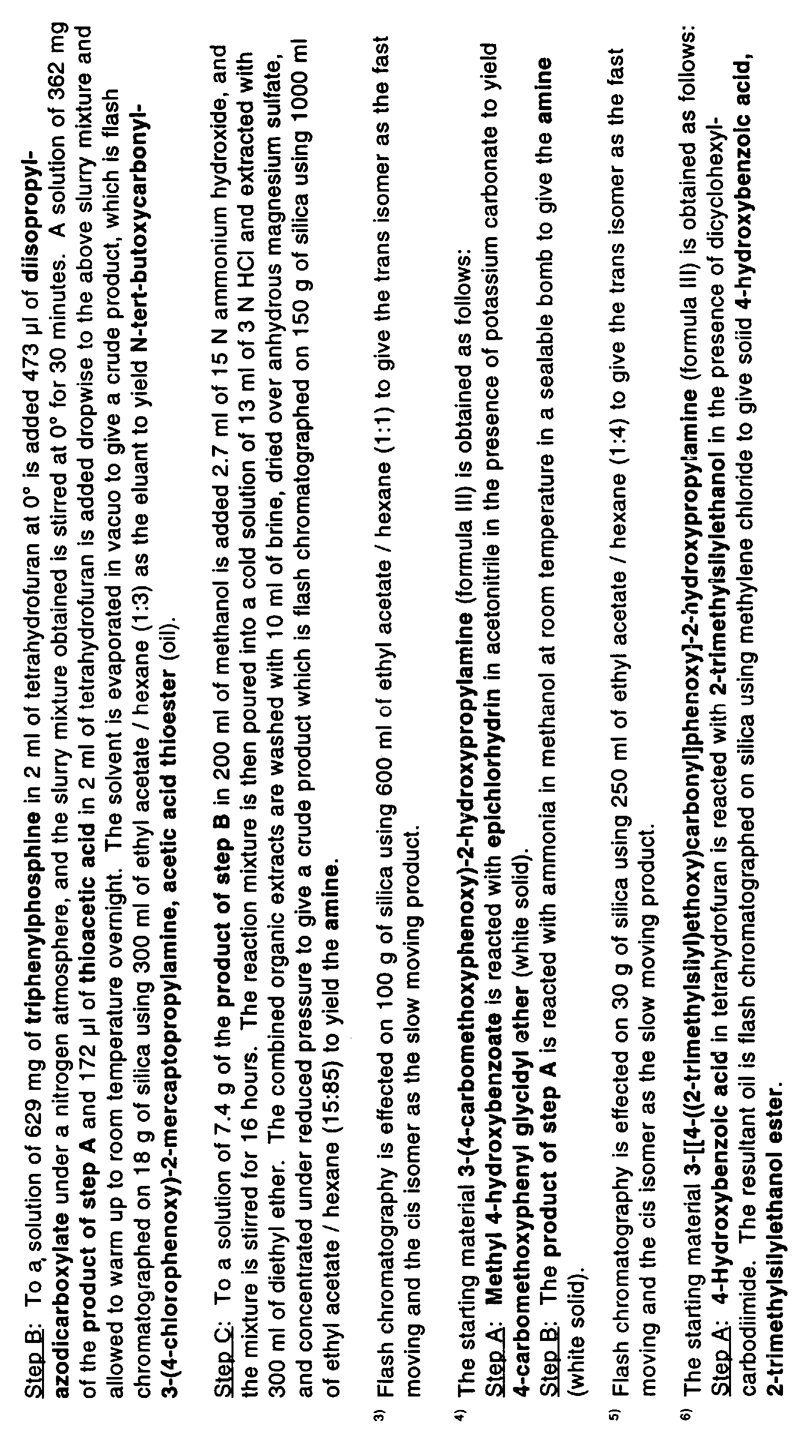

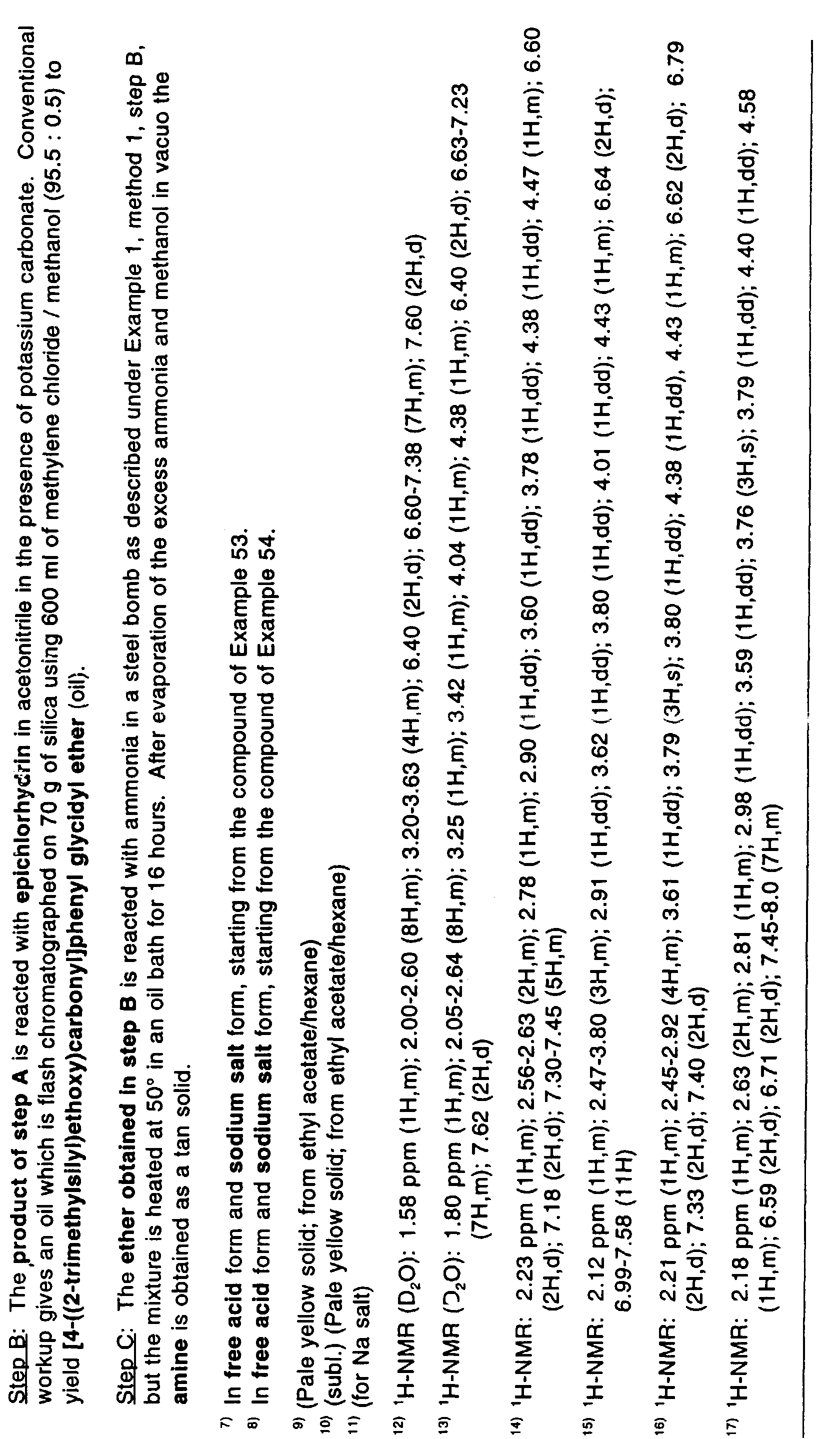

The compounds of formula I in free form or pharmacologically acceptable salt form as appropriate, hereinafter briefly named "the agents of the invention", exhibit pharmacological activity. In particular, they are hypoglycemic agents. They are thus indicated for use as pharmaceuticals, e.g. in the treatment of diabetes.

This is indicated in standard tests, e.g. in vitro by the L6 myocyte screen in a rat muscle cell line:

The L6 myocyte screen determines the direct effects of 1 to 300 µM concentrations of the agents on glucose utilization in the rat muscle cell line. Rat L6 myoblasts (American Type Culture Collection) are maintained in Dulbecco's modified Eagle's medium (DMEM, Gibco) containing 5 mM glucose and 10 % calf serum supplement. The L6 myoblasts are plated in 96-well microtiter plates at a density of 3000 cells/well. Differentiated myocytes are used in experiments on the 11th or 13th day after plating. All test agents are dissolved in 1 % dimethylsulfoxide (DMSO) in serum-free testing media. The DMSO has no effect on glucose utilization. Each experiment includes the following internal controls: water, glucose standard (300 mg/dl), media blank, media control, ciglitazone standard (30 µM) and an insulin dose-response curve (0.03 - 1 µM). All compounds are tested in quadruplicate in each experiment at concentrations of 1, 3, 10, 30,100 and 300 µM. The L6 cells are treated with test compound and DMEM containing 15 mM glucose for 20 hours. Glucose utilization is assessed by measurement of glucose remaining in the media using a glucose oxidase assay (Ciba-Corning #S1004B). The EC₅₀ is the concentration of the test agent estimated to produce 50 % of the maximum response versus control.

The antidiabetic activity is indicated in vivo e.g. in the ob/ob hypoglycemia test in male ob/ob mice, given 1 to 200 mg/kg of drug. The mice, approximately 2 to 3 months of age, weighing about 30 g, are kept in a room at a controlled ambient temperature of 22° C and a 12/12 hour light/dark cycle for at least seven days before the screen and then during testing. In the ob/ob screen, Purina rodent chow and water are available ad libitum. For each screen, mice are matched and placed in treatment groups (six mice per group) on the basis of initial blood glucose levels the day prior to screening (day 0). Animals are dosed once a day for three days with either vehicle (carboxymethylcellulose 0.5 %, with Tween-80 0.2 %) or test compound in vehicle. The animals receive 0.1 ml/10 g body weight administered orally by gavage. On day 1, blood glucose levels are sampled from the tip of the tail at 2 and 4 hours post-dose. On day 3, blood glucose levels are sampled at 2, 4 and 8 hours post-dose. Blood glucose levels are measured by the glucose oxidase method (YSI Model 27, Yellow Springs, Ohio). Behavioral observations are made on day 2 before dosing and on day 3 before the 4 hour post-dose sampling period. These observations are based on 10 indices of behavior and include diarrhea, mortality, lacrimation, locomotion, piloerection, twitches, tremors, body position, respiratory rate, and body temperature. They are selected from Irwin S., Comprehensive observational assessment: 1a. A systematic, quantitative procedure for assessing the behavioral and physiological state of the mouse, in Psychopharmacologica 12 (1968) 222-257.

Efficacy for glucose lowering is determined by comparing glucose levels of control animals to those of treated animals. Efficacy of 100 % represents the ability to normalize glucose levels to that of lean mice. The ED₅₀ value, calculated on day 3, is the amount of test agent estimated to produce 50 % of maximal efficacy in ability of the agent to normalize blood glucose levels.

The agents of the invention are therefore indicated for use as hypoglycemic agents, e.g. in the treatment of diabetes. The anti-diabetically effective dosage of the agents of the invention will vary depending e.g. on the particular agent employed, the mode of administration and the severity of the condition being treated. However, an indicated daily dosage is from about 50 mg to about 600 mg per day, preferably from about 100 mg to about 300 mg per day, or about 500 mg per day, conveniently administered e.g. in divided doses up to 4 times a day. The agents of the invention may be administered in a manner similar to known standards for the above uses.

It has been determined that the agent of Example 3, i.e. (±)-trans-2-[(4-chlorophenoxy)methyl]-7a-(3,4-dichlorophenyl)-2,3,7,7a-tetrahydropyrrolo[2,1-b]oxazol-5(6H)-one has an EC₅₀ of about 14 µM in the L6 myocyte test and about 62 mg/kg in the ob/ob hypoglycemia test. The preferred agent of the invention, the compound of Example 1, i.e. (-)-(trans)-2S-[(4-chlorophenoxy)methyl]-7a-(3,4-dichlorophenyl)-2,3,7,7a-tetrahydropyrrolo[2,1-b]oxazol-5(6H)-one has an EC₅₀ of about 10 µM in the L6 myocyte test and has significant activity from about 20 to 170 mg/kg in the ob/ob hypoglycemia test.

For the above use, the agents of the invention may be administered orally or parenterally, as such or admixed with conventional pharmaceutical diluents and carriers. They may be administered by any conventional route, in particular enterally, e.g. orally, in such forms as tablets, dispersible powders, granules, capsules, syrups and elixirs, and parenterally as solutions or emulsions. These pharmaceutical compositions may contain up to about 90 % of the active ingredient in combination with the carrier or adjuvant.

Capsules containing the ingredients indicated below may be prepared by conventional techniques and are useful in treating diabetes at a dose of one or two capsules two to four times a day:

| Ingredient | Weight (mg) |
|---|---|
| Compound of Example 1 | 250 |
| Lactose | 445 |
| Colloidal silicon | 50 |
| Stearic acid | 5 |
| | |
| Total | 750 |

The invention also provides pharmaceutical compositions comprising an agent of the invention together with at least one pharmaceutically acceptable carrier or diluent. Such compositions may be manufactured in conventional manner by mixing an agent of the invention together with at least one pharmaceutically acceptable carrier or diluent. Unit dosage forms contain, for example, from about 25 mg to about 500 mg of active substance.

## Claims

1. A compound of formula 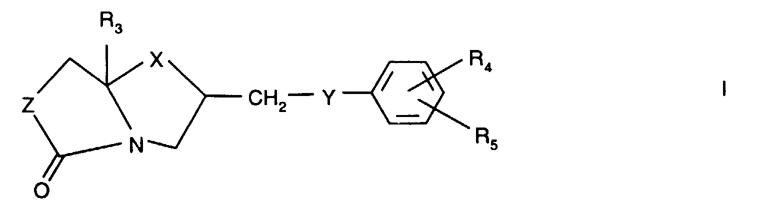 where
X and Y are each independently oxygen or sulfur;
Z is -CR₁R₂- or -CR₁R₂CH₂- where R₁ and R₂ are each independently hydrogen or C₁₋₄ alkyl;
R₃ is phenyl optionally mono- or disubstituted independently by halo, C₁₋₄ alkyl, C₁₋₄ alkoxy or trifluoromethyl; or is biphenyl; phenoxyphenyl; naphthyl; 9,10-dihydrophenanthryl; or pyridyl; and
R₄ and R₅ are each independently hydrogen, halo, C₁₋₄ alkyl, C₁₋₄ alkoxy, trifluoromethyl or -COOR₆ where R₆ is hydrogen, C₁₋₄ alkyl or triC₁₋₄alkylsilylC₁₋₄alkyl;
in free form or salt form as appropriate,

2. A compound according to claim 1 of formula I as defined in claim 1, in trans isomeric form with respect to the asymetrically substituted ring carbon atoms carrying the -CH₂Y- moiety and the R₃ moiety, respectively;
in free form or salt form as appropriate.

3. A compound according to claim 1 of formula I where
X and Y are both oxygen;
Z is -CR₁R₂- as defined in claim 1;
R₃ is phenyl optionally mono- or disubstituted independently by halo, C₁₋₄ alkyl, C₁₋₄ alkoxy or trifluoromethyl; or is 4-biphenyl; 4-phenoxyphenyl; 2-naphthyl; 9,10-dihydrophenanthryl; or 4-pyridyl;
R₄ is hydrogen, halo, C₁₋₄ alkyl, C₁₋₄ alkoxy, trifluoromethyl or -COOR₆ₐ where R₆ₐ is an alkali metal anion or C₁₋₄ alkyl; and
R₅ is hydrogen or halo;
or of formula I where
X and Y are both oxygen;
Z is -CH₂- or -C(CH₃)₂-;
R₃ is phenyl optionally mono- or disubstituted independently by fluorine or chlorine, or disubstituted by fluorine or chlorine and trifluoromethyl; or is 4-biphenyl; 4-phenoxyphenyl; 2-naphthyl; 9,10-dihydrophenanthryl; or 4-pyridyl;
R₄ is 4-chloro; and
R₅ is hydrogen.

4. A compound according to claim 1 of formula I where
X and Y are as defined in claim 1;
Z is -CH₂-, -C(CH₃)₂- or -CH₂CH₂-;
R₃ is phenyl optionally mono- or disubstituted independently by halo, methoxy or trifluoromethyl; 4-phenoxyphenyl; naphthyl; 9,10-dihydrophenanthryl; or pyridyl;
R₄ is hydrogen; halo; methoxy; trifluoromethyl; or COOR₆ where R₆ is hydrogen, methyl or (2-trimethylsilyl)ethyl; and
R₅ is hydrogen or halo;
in free form or salt form as appropriate.

5. A compound according to claim 1 of formula I where the substituents are as defined in claim 1, in free form or alkali metal salt form as appropriate.

6. (±)-trans-2-[(4-Chlorophenoxy)methyl]-7a-(3,4-dichlorophenyl)-2,3,7,7a-tetrahydropyrrolo[2,1-b]oxazol-5(6H)-one in free form or salt form as appropriate.

7. (-)-trans-2S-[(4-Chlorophenoxy)methyl]-7a-(3,4-dichlorophenyl)-2,3,7,7a-tetrahydropyrrolo[2,1-b]oxazol-5(6H)-one in free form or salt form as appropriate.

8. A process for preparing a compound according to claim 1 comprising
a) for the preparation of a compound of formula I where R₆ is other than hydrogen, reacting a compound of formula II 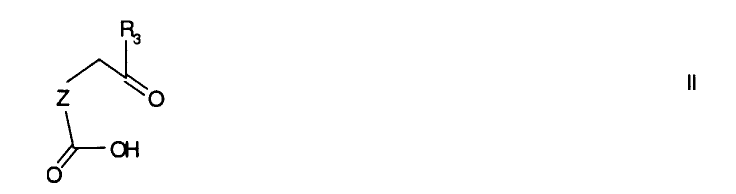 where R₃ and Z are as defined in claim 1, with a compound of formula III 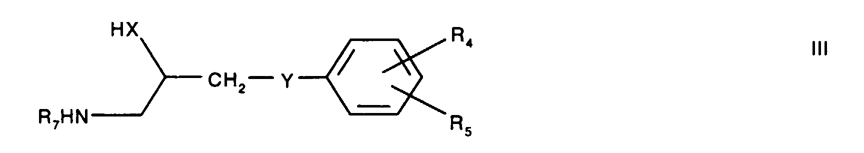 where X, Y, R₄ and R₅ are as defined in claim 1 and R₇ is hydrogen or an amine protecting group; or
b) for the preparation of a compound of formula I where R₆ is hydrogen, hydrolyzing a compound of formula I where R₆ is C₁₋₄ alkyl or triC₁₋₄alkylsilylC₁₋₄alkyl,
and recovering the resultant compound of formula I in free form or salt form as appropriate.

9. A pharmaceutical composition comprising a compound according to any one of claims 1 to 7 in free form or pharmacologically acceptable salt form as appropriate together with at least one pharmaceutically acceptable carrier or diluent.

10. A compound according to any one of claims 1 to 7 in free form or pharmacologically acceptable salt form as appropriate for use as a pharmaceutical, especially in the treatment of diabetes.

## Patentansprüche

1. Verbindung der Formel 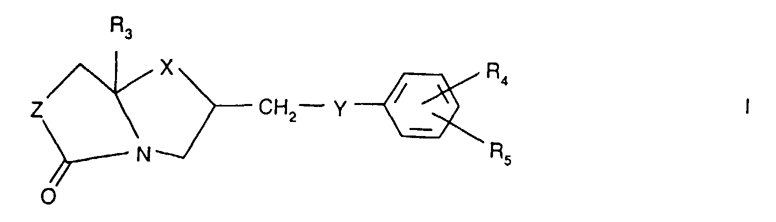 wobei
X und Y jeweils unabhängig Sauerstoff oder Schwefel sind,
Z -CR₁R₂- oder -CR₁R₂CH₂- ist, wobei R₁ und R₂ jeweils unabhängig Wasserstoff oder C₁₋₄-Alkyl sind,
R₃ Phenyl ist, das gegebenenfalls mono- oder unabhängig disubstituiert ist mit Halogen, C₁₋₄-Alkyl, C₁₋₄-Alkoxy oder Trifluormethyl, oder Biphenyl ist, Phenoxyphenyl, Naphthyl, 9,10-Dihydrophenanthryl oder Pyridyl, und
R₄ und R₅ jeweils unabhängig Wasserstoff, Halogen, C₁₋₄-Alkyl, C₁₋₄-Alkoxy, Trifluormethyl oder -COOR₆ sind,
wobei R₆ Wasserstoff, C₁₋₄-Alkyl oder tri-(C₁₋₄)-Alkylsikyl-(C₁₋₄)-alkyl ist,
nach Bedarf in freier oder Salz-Form.

2. Verbindung der Formel I nach Anspruch 1 in isomerer trans-Form bezüglich der asymmetrisch substituierten Ring-Kohlenstoffatome, die die -CH₂Y-Gruppe bzw. die R₃-Gruppe tragen, nach Bedarf in freier oder Salz-Form.

3. Verbindung der Formel I nach Anspruch 1, wobei
X und Y beide Sauerstoff sind,
Z -CR₁R₂- wie in Anspruch 1 definiert ist,
R₃ Phenyl ist, das gegebenenfalls mono- oder unabhängig disubstituiert ist mit Halogen, C₁₋₄-Alkyl, C₁₋₄-Alkoxy oder Trifluormethyl, oder 4-Biphenyl ist, 4-Phenoxyphenyl, 2-Naphthyl, 9,10-Dihydrophenanthryl oder 4-Pyridyl,
R₄ Wasserstoff, Halogen, C₁₋₄-Alkyl, C₁₋₄-Alkoxy, Trifluormethyl oder -COOR₆ₐ ist, wobei R₆ₐ ein Alkalimetall-Anion oder C₁₋₄-Alkyl ist, und
R₅ Wasserstoff oder Halogen ist,
oder der Formel I, wobei
X und Y beide Sauerstoff sind,
Z -CH₂- oder -C(CH₃)₂- ist,
R₃ Phenyl ist, das gegebenenfalls mono- oder unabhängig disubstituiert ist, mit Fluor oder Chlor, oder disubstituiert ist mit Fluor oder Chlor und Trifluormethyl, oder 4-Biphenyl ist, 4-Phenoxyphenyl, 2-Naphthyl, 9,10-Dihydrophenanthryl, oder 4-Pyridyl.
R₄ 4-Chlor ist, und
R₅ Wasserstoff ist.

4. Verbindung der Formel I nach Anspruch 1, wobei
X und Y wie in Anspruch 1 definiert sind,
Z -CH₂-, -C(CH₃)₂- oder -CH₂CH₂- ist,
R₃ Phenyl ist, das gegebenenfalls mono- oder unabhängig disubstituiert ist mit Halogen, Methoxy oder Trifluormethyl, 4-Phenoxyphenyl ist, Naphthyl, 9,10-Dihydrophenanthryl oder Pyridyl,
R₄ Wasserstoff, Halogen, Methoxy, Trifluormethyl oder COOR₆ ist, wobei R₆ Wasserstoff, Methyl oder (2-Trimethylsilyl)ethyl ist, und
R₅ Wasserstoff oder Halogen ist,
nach Bedarf in freier oder Salz-Form.

5. Verbindung der Formel I nach Anspruch 1, wobei die Substituenten wie in Anspruch 1 definiert sind, nach Bedarf in freier oder als Alkalimetallsalz-Form.

6. (±)-trans-2-[(4-Chlorphenoxy)methyl]-7a-(3,4-dichlorphenyl)-2,3,7,7a-tetrahydropyrrol[2,1-b]oxazol-5(6H)-on nach Bedarf in freier oder als Salz-Form.

7. (-)-trans-2S-[(4-Chlorphenoxy)methyl]-7a-(3,4-dichlorphenyl)-2,3,7,7a-tetrahydropyrrol[2,1-b]oxazol-5(6H)-on nach Bedarf in freier oder als Salz-Form.

8. Verfahren zur Herstellung einer Verbindung nach Anspruch 1, welches umfaßt:
a) zur Herstellung einer Verbindung der Formel I, bei der R₆ nicht Wasserstoff ist,
Umsetzen einer Verbindung der Formel II 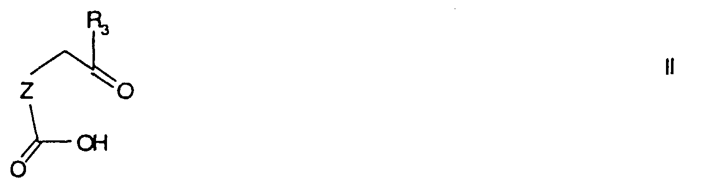 wobei R₃ und Z wie vorstehend definiert sind,
mit einer Verbindung der Formel III 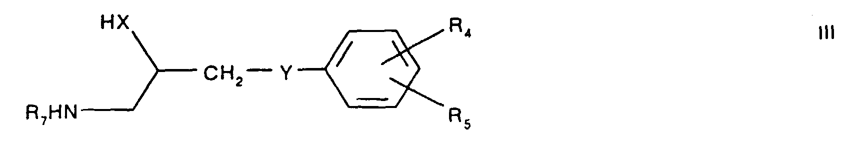 wobei X, Y, R₄ und R₅ wie in Anspruch 1 definiert sind, und R₇ Wasserstoff oder eine Amin-Schutzgruppe ist, oder
b) zur Herstellung einer Verbindung der Formel I, bei der R₆ Wasserstoff ist,
Hydrolysieren einer Verbindung der Formel I, bei der R₆ C₁₋₄-Alkyl oder tri-(C₁₋₄)-Alkylsilyl-(C₁₋₄)-alkyl ist, und Gewinnen der so erhaltenen Verbindung der Formel I nach Bedarf in freier oder Salz-Form.

9. Pharmazeutische Zusammensetzung, welche eine Verbindung nach einem der Ansprüche 1 bis 7 nach Bedarf in freier Form oder in Form eines pharmakologisch verträglichen Salzes zusammen mit mindestens einem pharmazeutisch verträglichen Träger oder Verdünnungsmittel enthält.

10. Verbindung nach einem der Ansprüche 1 bis 7 nach Bedarf in freier Form oder in Form eines pharmakologisch verträglichen Salzes, zur Verwendung als Arzneimittel, insbesondere bei der Behandlung von Diabetes.

## Revendications

1. Un composé de formule I 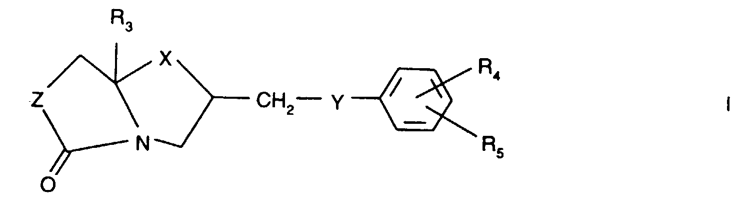 dans laquelle
X et Y signifient chacun indépendamment l'oxygène ou le soufre;
Z signifie -CR₁R₂- ou -CR₁R₂CH₂- où R₁ et R₂ signifient chacun indépendamment l'hydrogène ou (C₁₋₄)alkyle;
R₃ signifie un groupe phényle éventuellement mono- ou disubstitué indépendamment par halo, (C₁₋₄)alkyle, (C₁₋₄)alcoxy ou trifluorométhyle; ou signifie biphényle; phénoxyphényle; naphtyle; 9,10-dihydrophénanthryle; ou pyridyle; et
R₄ et R₅ signifient chacun indépendamment l'hydrogène, halo, (C₁₋₄)alkyle, (C₁₋₄)alcoxy, trifluorométhyle ou -COOR₆ où R₆ signifie l'hydrogène, (C₁₋₄)alkyle ou tri(C₁₋₄)alkylsilyl(C₁₋₄)alkyle;
sous forme libre ou sous forme d'un sel lorsque cela est approprié.

2. Un composé selon la revendication 1 de formule I tel que défini à la revendication 1, sous forme isomère trans par rapport aux atomes de carbone cycliques substitués asymétriquement portant respectivement le reste -CH₂Y- et le reste R₃ ;
sous forme libre ou sous forme d'un sel lorsque cela est approprié.

3. Un composé selon la revendication 1 de formule I où
X et Y signifient tous les deux l'oxygène;
Z signifie -CR₁R₂- tel que défini à la revendication 1;
R₃ signifie un groupe phényle éventuellement mono- ou disubstitué indépendamment par halo, (C₁₋₄)alkyle, (C₁₋₄)alcoxy ou trifluorométhyle; ou bien signifie 4-biphényle; 4-phénoxyphényle; 2-naphtyle; 9,10-dihydrophénanthryle; ou 4-pyridyle;
R₄ signifie l'hydrogène, halo, (C₁₋₄)alkyle, (C₁₋₄)alcoxy, trifluorométhyle ou -COOR₆ₐ où R₆ₐ signifie un anion de métal alcalin ou bien (C₁₋₄)alkyle; et
R₅ signifie l'hydrogène ou halo;
ou de formule I où
X et Y signifient tous les deux l'oxygène;
Z signifie -CH₂- ou -C(CH₃)₂-;
R₃ signifie un groupe phényle éventuellement mono- ou disubstitué indépendamment par le fluor ou le chlore ou disubstitué par le fluor ou le chlore et trifluorométhyle; ou bien signifie 4-biphényle; 4-phénoxyphényle; 2-naphtyle; 9,10-dihydrophénanthryle; ou 4-pyridyle;
R₄ signifie 4-chloro; et
R₅ signifie l'hydrogène.

4. Un composé selon la revendication 1 de formule I où
X et Y sont tels que définis à la revendication 1;
Z signifie -CH₂-, -C(CH₃)₂- ou -CH₂CH₂-;
R₃ signifie un groupe phényle éventuellement mono- ou disubstitué indépendamment par halo, méthoxy ou trifluorométhyle; 4-phénoxyphényle; naphtyle; 9,10-dihydrophénanthryle; ou pyridyle;
R₄ signifie l'hydrogène; halo; méthoxy; trifluorométhyle; ou COOR₆ où R₆ signifie l'hydrogène, un groupe méthyle ou (2-triméthylsilyl)éthyle; et
R₅ signifie l'hydrogène ou halo;
sous forme libre ou sous forme d'un sel lorsque cela est approprié.

5. Un composé selon la revendication 1 de formule I où les substitutants sont tels que définis à la revendication 1, sous forme libre ou sous forme d'un sel de métal alcalin lorsque cela est approprié.

6. La (±)trans-2-[(4-chlorophénoxy)méthyl]-7a-(3,4-dichlorophényl)-2,3,7,7a-tétrahydropyrrolo[2,1-b]oxazol-5(6H)-one sous forme libre ou sous forme d'un sel lorsque cela est approprié.

7. La (-)-trans-2S-[(4-chlorophénoxy)méthyl]-7a-(3,4-dichlorophényl)-2,3,7,7a-tétrahydropyrrolo[2,1-b]oxazol-5(6H)-one sous forme libre ou sous forme d'un sel lorsque cela est approprié.

8. Un procédé de préparation d'un composé selon la revendication 1, selon lequel
a) pour la préparation d'un composé de formule I où R₆ a une signification autre que l'hydrogène, on fait réagir un composé de formule II 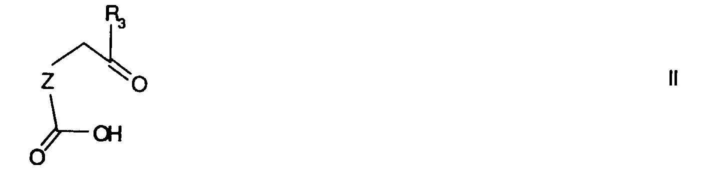 où R3 et Z sont tels que définis à la revendication 1, avec un composé de formule III 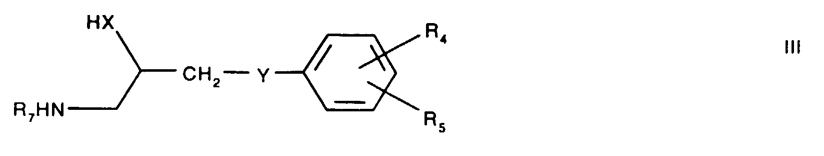 où X, Y, R₄ et R₅ sont tels que définis à la revendication 1 et R₇ signifie l'hydrogène ou un groupe protecteur d'une amine; ou bien
b) pour la préparation d'un composé de formule I où R₆ signifie l'hydrogène, on hydrolyse un composé de formule I où R₆ signifie un groupe (C₁₋₄)alkyle ou tri(C₁₋₄)alkylsilyl(C₁₋₄)alkyle,
et on récupère le composé résultant de formule I sous forme libre ou sous forme d'un sel lorsque cela est approprié.

9. Une composition pharmaceutique comprenant un composé selon l'une quelconque des revendications 1 à 7 sous forme libre ou sous forme d'un sel pharmacologiquement acceptable, lorsque cela est approprié, ensemble avec au moins un véhicule ou diluant pharmaceutiquement acceptable.

10. Un composé selon l'une quelconque des revendications 1 à 7 sous forme libre ou sous forme d'un sel pharmacologiquement acceptable , lorsque cela est approprié, pour une utilisation comme médicament, spécialement pour le traitement du diabète.
